(19) **Europäisches Patentamt**
**European Patent Office**
**Office européen des brevets**

(11) **EP 3 156 082 B1**

(12) **EUROPEAN PATENT SPECIFICATION**

(45) Date of publication and mention
of the grant of the patent:
**24.06.2020 Bulletin 2020/26**

(51) Int Cl.:
*A61L 27/10* (2006.01)  *C04B 35/22* (2006.01)
*C04B 35/20* (2006.01)  *C04B 33/24* (2006.01)
*C04B 35/622* (2006.01)  *C01B 33/24* (2006.01)
*C01B 33/22* (2006.01)

(21) Application number: **16002200.0**

(22) Date of filing: **13.10.2016**

(54) **PRODUCTION OF MONTICELLITE (CAMGSIO4) BASED BIOACTIVE CERAMICS FROM BORON WASTE**

HERSTELLUNG VON MONTICELLIT (CAMGSIO4) AUF DER BASIS VON BIOAKTIVEN KERAMIKEN AUS BORABFALL

PRODUCTION DE MONTICELLITE (CAMGSIO4) À BASE DE CÉRAMIQUES BIOACTIVES PROVENANT DE DÉCHETS DE BORE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.10.2015 TR 201512718**

(43) Date of publication of application:
**19.04.2017 Bulletin 2017/16**

(73) Proprietor: **Anadolu Universitesi Rektorlugu Eskisehir (TR)**

(72) Inventors:
- **KOROGLU, Levent**
  **26555 Eskisehir (TR)**
- **AYAS, Erhan**
  **26555 Eskisehir (TR)**

- **OVER, Derya**
  **26555 Eskisehir (TR)**
- **GUNEY, Yucel**
  **26555 Eskisehir (TR)**

(74) Representative: **Dericioglu, E. Korhan**
**Ankara Patent Bureau**
**Kavaklidere Mahallesi Bestekar Caddesi No: 10**
**06680 Cankaya, Ankara (TR)**

(56) References cited:
**EP-A1- 0 401 793     JP-A- 2005 289 701**

Remarks:
The file contains technical information submitted after the application was filed and not included in this specification

## Description

### Technical Field

The invention relates to biomaterials used in the health sector (medical industry) as a result of being generated by synthesizing raw material in the form of powder with powder metallurgy in biotechnology/biomedical technology field being an interdisciplinary art and technology branch.

The invention pertains to the area of ceramic biomaterials positively contributing to the bone regeneration. The ceramic based materials of the current invention are used as artificial bone in the production of various prostheses (orthopaedic implants), as implant material in the ocular implant implementation, in the antibacterial vaccination implementation, in gene transfer implementations, in cosmetic implementations, in bone, tooth and connection screws coverings as coating material.

### State of the Art

All of the synthetic or processed materials designed to partially or fully perform the functions of the tissue or organ of the body damaged or having lost its function, are called biomaterial.

The foremost characteristic of the biomaterials is that the continuity of the characteristics can be sustained without deteriorating in a biological medium.

The biomaterials partially or fully replace, support or treat the damaged tissue, organ or function of the body for a certain time period. In case a material is used on a person, this material is requested to be resistant to mechanical effects, chemical effects, be heat-resistant and radiation proof and have a bioavailability property.

The biomaterials are used to repair or regenerate the bone parts of the musculoskeletal system. The autologous bone grafts, in other words the bone grafts obtained from the patient himself/herself are widely used to fill the bone gravities and surgical reconstruction.

However, a limited number of bone supply is available and these procedures lead the patient to be subjected to further trauma for the graft to be obtained.

It is of significance in what way the mechanical properties of all biomaterials interacting with body tissues will show alteration.

Nowadays industrialization, space industry, developments in military technology and improvements in the orthopaedic surgery field significantly increased the use of biomaterials in the medical field. The most frequently used biomaterials can be classified under 3 headings as metallic, ceramic, and polymeric material.

The ceramics are generally known as inorganic, non-metallic materials. The basic practices of the ceramics in the orthopaedics field pertain to total hip and knee replacement. Otherwise, the ceramic materials can be used alone or together with other materials, ceramic based composites of osteogenic, osteoinductive or osteoconductive properties.

Furthermore, these ceramics can also be used as vessel for cells, growth factors, antibiotics and anti-cancer drugs. The use of bioceramic materials has decreased the abrasion rate of the components. The bioactivity of the ceramic materials is limited with osteoconductivity. The primary purpose of implanting the porous deformable ceramics to the bone is to provide the natural tissue replacement in the defective section.

The foremost advantage of the ceramics as to metals is that it can biologically interact with the host tissue. The use of bioceramic materials has decreased the abrasion rate of the components. The bioactivity of the ceramic is limited with osteoconductivity in case it does not carry cell or growth factor.

The bioceramics developed in the recent years; are separated into 3 groups as bioinert, bioactive and biocompatible (biodegradable). While bioinert ceramics form a mechanical connection with the tissue, bioactive ceramics interact by chemically connecting between the tissue and the implant. The biocompatible ceramics whereas replace the tissue.

The first generation ceramic materials, alumina and zirconia ceramics are generally accepted to be bioinert. One of the pilot implementations of the bioceramics is the use of high density and purity alumina as the traditional metallic femoral head in the hip prosthesis. Thereafter the ceramic materials have also been used as an acetabular cup. Excellent abrasion resistance rate, excellent corrosion resistance, good biocompatibility and high stability rates have been observed. This means that it was showing a significant advantage compared with classical polyethylene (PE) cups. Alumina is being used for approximately 20 years due to its low coefficient of friction and wear. Zirconia is one of the ceramic materials having the highest power suitable for medical use.

For the zirconia femoral heads articulated with an alumina insert in hip prosthesis, considerably low abrasion was reported under normal laboratory hip simulation conditions. This combination has entered the clinical use. While their biocompatibilities, high resistance to corrosion and abrasion form an advantage, their fragility, their difficulty of processing, their inelasticity are their most significant disadvantages.

Different to bioinert ceramics, the second generation ceramic materials are bioactive ceramics and their basic properties are that an appropriate surface for bone bonding and growth is provided.

Basically they are used as bone defect fillers. The resemblance between the bone mineral phase and their structural and surface properties is related with bonding with the bone with their good bioactive properties, without having any fibrous connective tissue interface present. Bioactive ceramics are biocompatible and osteoconductive materials.

The third generation ceramic materials are biocompatible materials designed to stimulate specific cellular response at molecular level. These materials can generate specific cellular response at molecular level. For biocompatible ceramics, the notion of bioactivity and biodegradability are in conjunction and while biologically absorbable materials can be bioactive, the exact opposite is also possible.

Lately the bone-graft interaction is intended to be improved with implementations at nano level. Bioactive glasses (BGS), glass-ceramics are basically glasses chemically containing $SiO_2$. Some of the $SiO_2$ groups have replaced calcium, phosphor or sodium oxides. Glass has increased the mechanical properties and biocompatibility of the ceramics used in the medical field. When these special types of ceramics are compared with Hydroxyapatite (HA), it has been seen that the osteogenic activity is increased. It has been observed that even small amounts of glass ionomers could bind the apatite particles efficiently. Glass ceramics cause a faster bone growth compared with HA. A direct connection was found between the mineralized layers formed on the surface of glass ceramics and the osteoblasts. The most significant disadvantage is that they are prone to chips and cracks. It is used for repairing or regenerating the bone tissue. The bond strength formed in certain conditions outgrows the endurance of its surrounding bone.

The bone consists of an inorganic scaffold and an inorganic matrix supporting and accommodating this scaffold. The inorganic scaffold of the bone basically consists of calcium and phosphate. While calcium and phosphate can be found in the structure of various living beings such as corals, it can also be chemically synthesized. The chemically synthesized ceramic (synthetic hydroxyapatite) can be in the form of natural calcium hydroxyapatite, calcium phosphate or a component of various rates of both crystals.

Due to their interconnected pore structures and biocompatibility, the hydroxyapatite (HA) ceramics are used as controlled drug release systems on implant coatings, bone tumors and the treatment of osteomyelitis. The advantage of these materials is that they are biocompatible and can be left in the tissue without the need of a second surgery at the end of the treatment due to their osteoconductive properties. Lately the clinical use of porous bioactive glasses instead of bones has increased. Bioactive glasses are silisium (Si), sodium (Na) and calcium (Ca) based and biocompatible, displaying a strong interface connection with the bone. Their bioactivities contribute to the formation of an apatite layer greatly similar to the mineral portion of the bone on the surface of the bioactive glasses. This apatite is characterized by the nanometric size, stoichiometric and/or non-stoichiometric and partial amorphous particles. The tissue adhesion rate changes dependent on the apatite formation rate and this is followed by a well-defined reaction sequence between the implanted material, the surrounding tissues and the physiologic liquids.

Alongside the above mentioned ceramic biomaterials, Si, Ca and P based 90% Bioglass Bioactive Strip Bone and Tooth Vaccine, (Ti), (N) and (0) based Titanium Nitrite Oxide-Coronary Stent, bioactive milling glass for Si, Ca, Na, P and 0 based cosmetic applications, Bioactive Tooth Glass used as 45S5 formula tooth filling, adhesive and treatment material, biocompatible polymer for antibacterial orthopeadic vaccines, Antibacterial Bioactive Glasses with S53P4 formula bioactive antibacterial composite material with patent application US8778378 consisting of porous calcium phosphate and bioactive glass are used.

Monticellite is an olivine group silicate mineral with the formula $CaMgSiO_4$, exhibiting vitreous and glossy properties. This silicate mineral exhibiting vitreous properties has been used in the production of bioactive ceramics. In the article Chen, X. et al., "Synthesis and Characteristics of Monticellite Bioactive Ceramic", J Mater Sci: Mater Med, 19, 1257-1263, 2008 referring to the said mineral, Monticellite ($CaMgSiO_4$) mono-phased ceramic has been successfully synthesized in sol-gel-derived monticellite powder tablets at 1480 °C heat for 6 hours.

Products sharing a similar quality (bioactivity, Si, Ca, Mg) with monticellite are akermanite, merwinite and diopside ceramics.

In the prior art, EP 0401793 A1 application discloses a ceramic material containing CaO and $SiO_2$ as main components which can be used for living hard tissue replacements and MgO as optional component does not comprise calcium phosphate and can form calcium phosphate-based compound as a result of contact with aqueous solution containing phosphorus. Moreover, it is also stated that this material may exhibit a high biological tendency and may be monticellite. Another application stated in prior art, JP 2005289701, discloses that the preliminary mixture containing 38-44% Si02, 20-27% MgO, 33-37% CaO and 1.5-3% boron by mass as additive material is heat treated between 850-1000°C and sintered monticellite crystals are obtained. The method disclosed in JP 2005289701, have lots of pretreatment steps like wet mixing, calcination etc. That means more energy consumption and lots of time requirement. The ceramic materials in both of EP 0401793 A1 and JP 2005289701 applications synthesized from commercial chemicals and that causes high cost.

The following studies have been performed in relation to these ceramics;

Wu, C. and Chang, J., "A Novel Akermanite Bioceramic: Preparation and Characteristics", Journal Of Biomaterials Applications, 21, 119-129, 2006.

Huang, Y. et al., "In Vitro and In Vivo Evaluation of Akermanite Bioceramics for Bone Regeneration", Biomaterials, 30, 5041-5048, 2009.

Ou, J. et al., "Preparation and In Vitro Bioactivity of Novel Merwinite Ceramic", Biomed. Mater., 3, 1-8, 2008.

Hafezi-Ardakani, M. et al., "Synthesis and Characterization of Nanocrystalline Merwinite (Ca3Mg(SiO4)2) via Sol-Gel Method", Ceramics International, 37, 175-180, 2011.

Iwata, N. Y. et al., "Preparation of Diopside with Apatite-Forming Ability by Sol-Gel Process Using Metal Alkoxide and Metal Salts", Colloids and Surfaces B: Biointerfaces, 33, 1-6, 2004.

In the state of the art and the first stage of the above stated studies, $HNO_3$ preferred as precipitant and many various starting materials such as tetraethyl orthosilicate [$(C_2H_5O_4Si$, TEOS], magnesium nitrate hexahydrate [$Mg(NO_3)_2.6H_2O$] and calcium nitrate tetrahydrate [$Ca(NO_3)_2.4H_2O$] are required in order to obtain monticellite starting powders. The sol-gel process carried out by using the said starting materials, the synthesis of the monticellite starting powders has been provided. For the sintering process, compact structures of Ø10x2.0 $mm^2$, 3x4x36 $mm^3$ and 4x8x10 $mm^3$ dimensions are obtained via uniaxial pressing process realized by applying 20 MPa pressure to the monticellite starting powders. Afterwards the production of single-phased monticellite (MgO wt. % = 25.76, Mg/Ca molar rate = 1) is provided under different temperatures such as 1350, 1400 and 1480°C via sintering processes lasting for 6 hours.

In the synthesis method used under the light of the above given information, the use of a wide range of starting materials with complex compounds, the preference of high sintering temperatures, the preparation of colloidal solutions and gels according to the sol-gel process, the removal and drying of solvents by evaporating are among the significant factors increasing the product cost.

Additionally, the said multi-step sol-gel process, the required pressing process and the long sintering periods lasting for many hours, besides increasing the total product cost, also increase the production time, prevents the production process to be functional and cause significant losses in the high productivity value required for mass production.

Due to the aforementioned disadvantages, the single-step synthesis method, subject of the invention has been developed.

## The Problems Aimed to be Resolved by the Invention

The purpose of the invention is to obtain biomaterials used in the health sector wherein a wide range of starting materials with complex compounds are not used, the production time is shortened by a single-step synthesis process, the raw materials in the form of powder are synthesized and produced in the biotechnology/biomedical technology field.

Another purpose of the invention is to decrease the energy requirement during the production by obtaining Monticellite phase obtainable at temperatures 1300°C and above, at lower temperatures such as 800°C - 900 °C using synthetic CaO, MgO and $SiO_2$ powders.

Another purpose of the invention is to provide a functional production process and the production of Monticellite ($CaMgSiO_4$) based bioactive ceramics providing gain in the high producibility value required for mass production.

Another purpose of the invention is to provide the synthesis of Monticellite ($CaMgSiO_4$) based bioactive ceramics from the wastes emerging during mining activities.

Another purpose of the invention is to lower the cost of the starting material and to make use of the waste materials by using the waste emerging during mining activities.

Another purpose of the invention is to lower the waste storage cost, to abate environmental pollution, to prevent the wastes to reach people via underground-surface waters and the respiratory tract and cause health problems by using the boron waste emerging during mining activities.

## Disclosure of the Invention

The invention pertains to biomaterials, as disclosed in claim 1, used in the health sector (medical industry) as a result of being generated by synthesizing raw material in the form of powder with powder metallurgy in biotechnology/biomedical technology field being an interdisciplinary art and technology branch.

The synthesis method developed is a single-step synthesis method, as disclosed in claim 2, wherein a wide range of starting materials with complex compounds are not used and by using the boron waste emerging upon mining activities as starting material. Thus, the biomaterial obtained

upon synthesis, is Monticellite ($CaMgSiO_4$) based bioactive ceramic material comprising monticellite, calcium borosilicate and strontium silicate.

The boron waste powder expressed as waste upon mining activities and not being used are put to use as starting raw material. These starting powders are directly taken into sintered alumina crucibles without being subjected to any kind of grinding, preparing solution and gel, drying and pressing processes and are subjected to heat treatment in a simple oven for less time and at much lower temperature than the synthesis temperature used in the prior art.

In order to produce monticellite based bioactive ceramic material, boron waste powders containing mainly CaO, MgO, $Na_2O$, $B_2O_3$ and $SiO_2$ compounds, high amounts of dolomite [$CaMg(CO_3)_2$], calcite[$CaCO_3$], tincalconit [$Na_2B_4O_7$-

$5H_2O$], free quartz [$SiO_2$] phases and small amounts of strontium carbonate [$SrCO_3$] phase are subjected to heat treatment for 0-3 hours between 600-900°C temperature range at 1-30°C/min. heating rate within sintered alumina crucibles in powered high temperature ovens. After the heat treatment it is subjected to sudden cooling process by being taken out of the oven.

Realizing the phase transformations together with the heat treatment and completing the single-step production process of the monticellite based bioactive ceramic materials, the monticellite based bioactive ceramic material is synthesized. The **monticellite** ($CaMgSiO_4$) during the heat treatment performed for the synthesis of the aforementioned monticellite based bioactive ceramic material,

$$SiO_2 + CaMg(CO_3)_2 \rightarrow CaMgSiO_4 + 2CO_2(g) \qquad \text{(Equation 1.)}$$

**calcium borosilicate** ($Ca_2B_2SiO_7$) as a result of the reaction of tincalconite, calcite and quartz phases,

➢

$$Na_2B_4O_7 - 5H_2O \rightarrow Na_2B_4O_7 + 5H_2O \qquad \text{(Equation 2.)}$$

➢

$$CaCO_3 \rightarrow CaO + CO_2 \qquad \text{(Equation 3.)}$$

➢

$$Na_2B_4O_7 + 4CaO + 2SiO_2 \rightarrow 2Ca_2B_2SiO_7 + Na_2O \qquad \text{(Equation 4.)}$$

and strontium silicate ($Sr_3SiO_5$) phases are formed as a result of the reaction between strontium and quartz

➢

$$3SrCO_3 + SiO_2 \rightarrow Sr_3SiO_5 + 3CO_2(g) \qquad \text{(Equation 5.)}$$

It has been stated in the studies made and the information in the prior art that these phases occurring upon the heat treatment are also biocompatible and bioactive.

In the biotechnology practices the bioactive materials are used as powder or granule as direct product. Therefore, heat treatment is applied to the monticellite based bioactive ceramic material. Following the heat treatment, the monticellite based bioactive ceramics obtained from crucibles are milled in an agate mortar and made ready for the practice for the agglomerates to break and no contamination to occur in the meantime.

In the aforementioned synthesis method, the crucibles wherein the boron waste powders will be placed, are $Al_2O_3$ based crucibles.

In an embodiment of the invention, the powder form waste (120 g) inside the alumina crucible (approximate base diameter: 100 mm, upper diameter: 150 mm, height: 190 mm) placed inside a box and/or tube shaped oven inside and/or on the refractory base has been subjected to heat treatment at a temperature range of 600-900°C with a heating speed of 1°C/min-30°C/min for 1 hour and cooled at room temperature. After the heat treatment, the monticellite based bioactive ceramic material obtained inside the crucible as bulk, are milled inside the agate mortar for approximately 5 minutes.

The Monticellite based bioactive ceramic material obtained as powder after the milling process is made ready for the implementation as final product.

An in vitro bioactivity test has been performed for the monticellite based bioactive ceramic material obtained as a result of the synthesis method subject to the invention.

In order to make use of the bioactivity of the monticellite based bioactive ceramics obtained upon the heat treatment, the pellet samples brought to 16 Ø x 7 mm dimensions were held in a biocompatible environment representing the human body (37°C and pH=7.40 conditions) for 5, 15 and 25 days inside simulated body fluid (SBF) simulating blood and body fluids. It has been determined that the samples reacted with SBF after the test and as from 15 days apatite layers, indication of bioactivity, were formed as a result of precipitation of calcium-phosphate on their surfaces.

The monticellite based bioactive ceramics obtained as a result of functional and low cost heat treatment can be used in the materials to be used for bone regeneration, biomedical applications such as trauma surgery, chin and face surgery, dental surgery, orthodontics surgery, endodontics surgery, ophthalmology, neurosurgery, antibacterial vaccines, implant, implant coating, gene transfer and cosmetics.

In addition to this, the monticellite based bioactive ceramics obtained by the synthesis method subject to the invention, compared to prior arts provide many advantages such as providing cost-effective raw material access, not requiring substructure equipment reaching high financial figures and involving a quite functional single-step production process by gaining time and energy.

## Claims

1. A biomaterial used in the medical field generated by synthesizing raw material in the form of powder with powder metallurgy in biotechnology/biomedical technology field **characterized by** being a monticellite ($CaMgSiO_4$) based bioactive ceramic material comprising monticellite, calcium borosilicate and strontium silicate and synthesized by using boron waste powders as starting material containing mainly CaO, MgO, $Na_2O$, $B_2O_3$ and $SiO_2$ compounds, high amounts of dolomite [$CaMg(CO_3)_2$], calcite[$CaCO_3$], tincalconit [$Na_2B_4O_7$-$5H_2O$], free quartz [$SiO_2$] phases and small amounts of strontium carbonate [SrCO3].

2. A single-step synthesis method of the biomaterial according to Claim 1, **characterized by** comprising the below steps:

   • Heating boron waste powders containing mainly CaO, MgO, $Na_2O$, $B_2O_3$ and $SiO_2$ compounds, high amounts of dolomite [$CaMg(CO_3)_2$], calcite[$CaCO_3$], tincalconit [$Na_2B_4O_7$-$5H_2O$], free quartz [$SiO_2$] phases and small amounts of strontium carbonate [$SrCO_3$] phase for 0-3 hours between 600-900°C temperature range at 1-30°C/min heating rate within sintered alumina crucibles in powered high temperature oven and,
   • Sudden cooling the sintered raw materials by taking out from the powered high temperature oven.

3. A synthesis method according to claim 2, wherein the biomaterial in bulk form obtained after the said sudden cooling is followed by milling the biomaterial in agate mortar, thereby preventing contamination.

## Patentansprüche

1. Biomaterial zur Verwendung im Bereich der Medizin, das durch die Synthese von pulverförmigem Rohmaterial mit Pulvermetallurgie im Bereich der Biotechnologie/Biomedizintechnik erzeugt wird, **gekennzeichnet durch** ein bioaktives keramisches Material auf der Basis von Monticellit ($CaMgSiO_4$) ist, das Monticellit, Kalziumborsilikat und Strontiumsilikat umfasst und unter Verwendung von Bor-Abfallpulvern als Ausgangsmaterial synthetisiert werden, die hauptsächlich CaO, MgO, $Na_2O$, $B_2O_3$ und $SiO_2$ Verbindungen, hohe Anteile an Dolomit [$CaMg(CO_3)_2$], Kalzit [$CaCO_3$], Tinkalkonit [$Na_2B_4O_7$-$5H_2O$], freie Quarz [$SiO_2$] Phasen und geringe Anteile an Strontiumkarbonat [$SrCO_3$] enthalten.

2. Einzelschritt-Syntheseverfahren des Biomaterials nach Anspruch 1, **gekennzeichnet durch** die nachfolgenden Schritte umfasst:

   • Erhitzen von Bor-Abfallpulvern, die hauptsächlich CaO, MgO, $Na_2O$, $B_2O_3$ und $SiO_2$ Verbindungen, hohe Anteile an Dolomit [$CaMg(CO_3)_2$], Kalzit [$CaCO_3$], Tinkalkonit [$Na_2B_4O_7$-$5H_2O$], freie Quarz [$SiO_2$] Phasen und geringe Anteile an Strontiumkarbonat [$SrCO_3$] Phase enthalten, für 0-3 Stunden im Temperaturbereich von 600-900°C bei einer Heizrate von 1-30°C/min in gesinterten Aluminiumoxidtiegeln in einem betriebenen Hochtemperaturofen und,
   • Plötzliche Abkühlung der gesinterten Rohstoffe durch Entnahme aus dem betriebenen Hochtemperaturofen.

3. Syntheseverfahren nach Anspruch 2, wobei das Biomaterial in loser Form, das nach der genannten plötzlichen Abkühlung erhalten wird, gefolgt wird von einem Zerkleinern des Biomaterials in Achatmörser, dadurch wird eine Kontamination verhindert.

**Revendications**

1. Biomatériau utilisé dans le domaine médical généré par la synthèse de la matière première sous forme de poudre avec la métallurgie des poudres dans le domaine de la biotechnologie/technologie biomédicale **caractérisé par le fait qu'**il est un matériau céramique bioactif à base de monticellite ($CaMgSiO_4$) comprenant de la monticellite, du borosilicate de calcium et du silicate de strontium et synthétisé en utilisant des poudres de déchets de bore comme matière première contenant principalement des composés de CaO, MgO, $Na_2O$, $B_2O_3$ et $SiO_2$, de grandes quantités de dolomite [$CaMg(CO_3)_2$], de calcite [$CaCO_3$], de tincalconite [$Na_2B_4O_7$-$5H_2O$], de phases de quartz libre [$SiO_2$] et de petites quantités de carbonate de strontium [SrCO3].

2. Méthode de synthèse en une seule étape du biomatériau selon la revendication 1, **caractérisée par le fait qu'**elle comprend les étapes suivantes :

   • Chauffage des poudres de déchets de bore contenant principalement des composés de CaO, MgO, $Na_2O$, $B_2O_3$ et $SiO_2$, de grandes quantités de dolomite [$CaMg(CO_3)_2$], de calcite [$CaCO_3$], de tincalconite [$Na_2B_4O_7$-$5H_2O$], de phases de quartz libre [$SiO_2$] et de phase de petites quantités de carbonate de strontium [$SrCO_3$] pendant 0 - 3 heures dans une plage de température de 600 - 900°C à une vitesse de chauffage de 1 - 30°C/min dans des creusets d'alumine frittée dans un four électrique à haute température et,
   • Refroidissement brusque des matières premières frittées en les sortant du four électrique à haute température.

3. Méthode de synthèse selon la revendication 2, dans lequel le biomatériau en vrac obtenu après ledit refroidissement brusque est suivi par le broyage du biomatériau dans un mortier d'agate, ce qui empêche la contamination.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8778378 B
- EP 0401793 A1
- JP 2005289701 B

**Non-patent literature cited in the description**

- **CHEN, X. et al.** Synthesis and Characteristics of Monticellite Bioactive Ceramic. *J Mater Sci: Mater Med,* 2008, vol. 19, 1257-1263
- **WU, C. ; CHANG, J.** A Novel Akermanite Bioceramic: Preparation and Characteristics. *Journal Of Biomaterials Applications,* 2006, vol. 21, 119-129
- **HUANG, Y. et al.** Vitro and In Vivo Evaluation of Akermanite Bioceramics for Bone Regeneration. *Biomaterials,* 2009, vol. 30, 5041-5048
- **OU, J. et al.** Preparation and In Vitro Bioactivity of Novel Merwinite Ceramic. *Biomed. Mater.,* 2008, vol. 3, 1-8
- **HAFEZI-ARDAKANI, M. et al.** Synthesis and Characterization of Nanocrystalline Merwinite (Ca3Mg(SiO4)2) via Sol-Gel Method. *Ceramics International,* 2011, vol. 37, 175-180
- **IWATA, N. Y. et al.** Preparation of Diopside with Apatite-Forming Ability by Sol-Gel Process Using Metal Alkoxide and Metal Salts. *Colloids and Surfaces B: Biointerfaces,* 2004, vol. 33, 1-6